(19) Europäisches Patentamt
European Patent Office
Office européen des brevets

(11) **EP 2 980 053 B1**

(12) **EUROPEAN PATENT SPECIFICATION**

(45) Date of publication and mention
of the grant of the patent:
**21.02.2018 Bulletin 2018/08**

(51) Int Cl.:
*C07C 5/48* *(2006.01)*          *C07C 11/167* *(2006.01)*
*B01J 23/88* *(2006.01)*        *C07B 61/00* *(2006.01)*
*B01J 23/90* *(2006.01)*

(21) Application number: **14774257.1**

(22) Date of filing: **26.03.2014**

(86) International application number:
**PCT/JP2014/058643**

(87) International publication number:
**WO 2014/157390 (02.10.2014 Gazette 2014/40)**

(54) **BUTADIENE PRODUCTION METHOD**

BUTADIENHERSTELLUNGSVERFAHREN

PROCÉDÉ DE PRODUCTION DE BUTADIÈNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.03.2013 JP 2013067278**

(43) Date of publication of application:
**03.02.2016 Bulletin 2016/05**

(73) Proprietor: **Asahi Kasei Kabushiki Kaisha
Chiyoda-ku
Tokyo 101-8101 (JP)**

(72) Inventors:
• **AKAGISHI, Kenji
Tokyo 101-8101 (JP)**
• **YANAGI, Hiroyuki
Tokyo 101-8101 (JP)**

• **WATANABE, Mamoru
Tokyo 101-8101 (JP)**
• **YOSHINO, Fukiko
Tokyo 101-8101 (JP)**
• **MIDORIKAWA, Hideo
Tokyo 101-8101 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
JP-A- 2011 219 366     JP-A- 2012 067 046
JP-A- 2012 067 048     JP-A- 2012 072 076
JP-A- 2012 092 092

**Description**

Technical Field

The present invention relates to a process for producing butadiene.

Description of the Related Art

Background Art

Processes for producing 1,3-butadiene (hereinafter sometimes simply referred to as "butadiene") by an oxidative dehydrogenation reaction of n-butene and oxygen using a catalyst are well known.

Examples of the reaction process employed in this oxidative dehydrogenation reaction include processes such as fixed bed, fluidized bed, and moving bed reaction processes. Among these, in a fixed bed reaction process, since the fluidized state of the gas is close to the extrusion flow, such a process has the advantage that reaction yield can be increased, and is thus frequency employed industrially. However, a fixed bed reaction process has low thermal conductivity, and is thus not suited for exothermic reactions or endothermic reactions that require the removal or addition of heat. In particular, in a strongly exothermic reaction like an oxidative dehydrogenation reaction of n-butene, the temperature increases suddenly, so that the reaction can become difficult to control, which can lead to a runaway reaction. In addition, such a sudden increase in temperature can damage the catalyst, so that there is also the problem of premature degradation.

In contrast, in a fluidized bed reaction process, the catalyst particles flow vigorously inside the reactor, so there are the advantages that (1) since thermal conductivity is high, the reaction can be inhibited from progressing too much by maintaining the temperature in the reactor at a roughly uniform level even during reactions that involve a large amount of heat being produced or absorbed, and (2) since localized accumulation of energy is suppressed, a raw material gas that is in the explosive range can be reacted, which allows productivity to be improved by increasing the raw material concentration. Therefore, a fluidized bed reaction process is suited to the oxidative dehydrogenation reaction of n-butene, which is a very exothermic reaction. For example, the oxidative dehydrogenation reaction for synthesizing butadiene from n-butene produces about 30 kcal/mol of heat.

Examples of various reaction processes described above include the following patent documents. Patent Document 1 discloses a process for producing a gas that includes a conjugated diene by feeding a mixed gas of a raw material gas including a monoolefin having a carbon atom number of 4 or more and a molecular oxygen-containing gas to a fixed bed reactor having a catalyst. This document states that stable operation can be realized by suppressing the catalyst pulverization ratio after a predetermined reaction period to 40% by mass or less. Further, Patent Document 2 describes a process for producing butadiene from n-butene in a fluidized bed reactor in which n-butene and oxygen are present. This document discloses that, after performing a butadiene synthesis reaction for a predetermined period, regeneration is performed by stopping operation of the apparatus, and calcining all or a part of the catalyst to which organic matter has adhered as a result of being used in the reaction, and then the regenerated catalyst is again fed into the reaction.

Patent document 3 also discloses a process for the preparation of butadiene.

Citation List

Patent Document

    Patent Document 1: Japanese Patent Laid-Open No. 2012-46509
    Patent Document 2: Japanese Patent Laid-Open No. 2012-67047
    Patent Document 3: JP201292092

Summary of Invention

Technical Problem

In a conventional process, the regeneration of a catalyst to which organic matter is adhered is performed based on a so-called batch process, in which the regeneration is carried out after temporarily stopping operation of the reactor. A batch process is not desirable from a productivity viewpoint, since butadiene production has to be interrupted each time catalyst regeneration is carried out. Accordingly, there is a need to establish a continuous-type catalyst regeneration process that is capable of regenerating a catalyst while continuing operation of the reactor.

However, based on detailed investigations by the present inventors, it was learned that in the reaction for producing butadiene from n-butene, the amount of organic matter adhered to the catalyst exponentially increases with reaction time. It was also learned that although the amount of organic matter adhered to the catalyst is very slight for a certain time after the reaction, after the certain time, organic matter rapidly adheres to the catalyst, and moreover, the rate of adherence increases as the amount of organic matter adhered becomes greater.

Thus, since the adherence of organic matter to the catalyst exhibits a peculiar behavior, it is more difficult with a conventional process to regenerate the catalyst as the reactor is operating for a longer time. Consequently, the reactor has to be temporarily stopped in order to regenerate the catalyst.

It is an object of the present invention, which was created in view of the above-described problems, to provide a butadiene production process that is capable of suppressing a sudden increase in organic matter adhered to the catalyst that occurs with the reaction.

Solution to Problem

As a result of diligent research to solve the above-described problems, the present inventors discovered that by controlling the amount of the regenerated catalyst fed to a fluidized bed reactor at a predetermined ratio or more based on the amount of a monoolefin having 4 carbon atoms that is fed to the fluidized bed reactor, the sudden increase in organic matter adhered to the catalyst that occurs with the reaction can be suppressed, thereby completing the present invention.

Namely, the present invention is directed to the following.

[1] A process for producing butadiene, including:

a first step of producing butadiene by a vapor-phase catalytic oxidation reaction in the presence of a catalyst by feeding a monoolefin having a carbon number of 4 to a fluidized bed reactor; and
a second step of removing a part of the catalyst from the fluidized bed reactor while the vapor-phase catalytic oxidation reaction is proceeding, regenerating the catalyst removed from the fluidized bed reactor, and feeding the regenerated catalyst to the fluidized bed reactor while the vapor-phase catalytic oxidation reaction is proceeding,
wherein a ratio of an amount (kg/hr) of the regenerated catalyst fed based on an amount (kg/hr) of the monoolefin having a carbon number of 4 fed is 0.3% or more.

[2] The process for producing butadiene according to [1], wherein the ratio of the amount (kg/hr) of the regenerated catalyst fed based on the amount (kg/hr) of the monoolefin having a carbon number of 4 fed is 0.3 to 100%.
[3] The process for producing butadiene according to [1] or [2], wherein the catalyst includes a metal oxide containing at least Mo, Bi, and Fe.
[4] The process for producing butadiene according to any one of [1] to [3], wherein the catalyst includes a metal oxide represented by the following empirical formula (I),

$$Mo_{12}Bi_pFe_qA_aG_bJ_cL_dE_eO_x \qquad (I)$$

wherein A represents one or more elements selected from the group consisting of nickel and cobalt, G represents one or more elements selected from the group consisting of alkali metal elements, J represents one or more elements selected from the group consisting of magnesium, calcium, strontium, barium, zinc, and manganese, L represents one or more elements selected from the group consisting of rare earth elements, E represents one or more elements selected from the group consisting of chromium, indium, and gallium, $0.1 \leq p \leq 5$, $0.5 \leq q \leq 8$, $0 \leq a \leq 10$, $0.02 \leq b \leq 2$, $0 \leq c \leq 5$, $0 \leq d \leq 5$, $0 \leq e \leq 5$, and x denotes a number of atoms of oxygen required to satisfy a valence requirement of the other elements that are present in the metal oxide.
[5] The process for producing butadiene according to any one of [1] to [4], wherein the catalyst includes a support, and the support includes one or more selected from the group consisting of silica, alumina, titania, and zirconia.
[6] The process for producing butadiene according to any one of [1] to [4], wherein the catalyst includes a support, and the support is produced from two or more silica sols having a different silica average particle size.

Advantageous Effects of Invention

According to the process for producing butadiene according to the present invention, a sudden increase in organic matter adhered to the catalyst that occurs with the reaction can be suppressed. Consequently, butadiene can be stably produced at a high yield continuously over a long period without having to stop production of butadiene to regenerate the catalyst.

Brief Description of Drawings

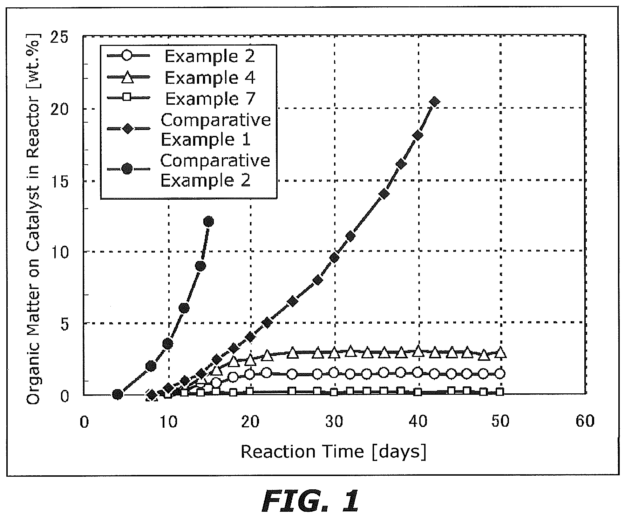

FIG. 1 is a graph illustrating the amount of organic matter adhered to the catalyst in the reactor with respect to reaction time.

Description of Embodiments

A preferred embodiment for carrying out the present invention (hereinafter, simply referred to as "present embodiment") will now be described in more detail. However, the present invention is not limited to the following present embodiment. Various modifications may also be made within the scope of the present invention.

The process for producing butadiene according to the present embodiment is a process in which butadiene is produced from a monoolefin having 4 carbon atoms by a vapor-phase catalytic oxidation reaction in a fluidized bed reactor. This process includes a first step of producing butadiene by a vapor-phase catalytic oxidation reaction (hereinafter, sometimes simply referred to as "reaction") in the presence of a catalyst by feeding a monoolefin having 4 carbon atoms to a fluidized bed reactor, and a second step of removing a part of the catalyst from the fluidized bed reactor while the vapor-phase catalytic oxidation reaction is proceeding, regenerating the catalyst removed from the fluidized bed reactor, and feeding the regenerated catalyst to the fluidized bed reactor while the vapor-phase catalytic oxidation reaction is proceeding. In this process, a ratio of the amount (kg/hr) of the regenerated catalyst fed to the amount (kg/hr) of the monoolefin having 4 carbon atoms fed (hereinafter, sometimes simply referred to as "regeneration ratio") is 0.3% or more. The regeneration ratio is calculated based on the following formula.

$$\text{Regeneration ratio (\%)} = \text{(amount (kg/hr) of regenerated catalyst fed to}$$

$$\text{fluidized bed reactor)/(amount (kg/hr) of monoolefin having 4 carbon atoms fed to}$$

$$\text{fluidized bed reactor)} \times 100$$

The process for calculating the amount of the monoolefin having 4 carbon atoms fed and the amount of the regenerated catalyst fed will now be described. The amount of the monoolefin having 4 carbon atoms fed and the amount of the regenerated catalyst fed are represented in terms of mass (kg) per unit time (hr). Since the amount of the monoolefin having 4 carbon atoms fed to the fluidized bed reactor while the vapor-phase catalytic oxidation reaction is proceeding fluctuates due to various factors, it is desirable to take an average value for a given predetermined period as the amount of the monoolefin having 4 carbon atoms fed. Similarly, since the amount of the catalyst to be regenerated can frequently change, it is desirable to take an average value for a given predetermined period as the amount of the regenerated catalyst fed. In the present embodiment, an average value for 50 days of reaction is employed. For example, if the total amount of the monoolefin having 4 carbon atoms fed for 50 days (1,200 hours) from the start of reaction was 1,200 kg, the amount of the monoolefin having 4 carbon atoms fed is calculated as 1,200 (kg)/1,200 (hr) = 1.0 (kg/hr). Similarly, if the total amount of the regenerated catalyst fed for 50 days (1,200 hours) from the start of reaction was 600 kg, the amount of the regenerated catalyst fed is calculated as 600 (kg)/1,200 (hr) = 0.5 (kg/hr).

Further, if the reaction time is less than 50 days, or if the reaction time calculated in terms of a 50 day period is a fraction, the average value for that period may be employed. For example, if the total reaction time is 30 days (720 hours), the average value of the 30 day period is employed. On the other hand, if the total reaction time is 80 days (1,920 hours), the reaction time is divided into a 50 day period and a 30 day period, and the average value of each period is calculated. Further, in the present embodiment, "the start of reaction" is defined as the point when feed of the monoolefin having 4 carbon atoms to the fluidized bed reactor is started.

[1] Butadiene production process

(1) Raw material

In the process for producing butadiene according to the present embodiment, the raw material of the butadiene is a monoolefin having 4 carbon atoms. Preferably, the raw material is n-butene (meaning 1-butene, 2-butene, as well as a mixture thereof). Usually, a gas containing n-butene (hereinafter, sometimes referred to as "n-butene-containing gas") in which n-butene has been diluted with another component can be used. In addition, it is preferred that molecular oxygen is present in the fluidized bed reactor with the monoolefin having 4 carbon atoms. Usually, a gas containing molecular oxygen (hereinafter, sometimes referred to as "oxygen-containing gas") in which molecular oxygen has been diluted with another component can be used.

The n-butene-containing gas does not have to include n-butene at a high purity, an arbitrary mixture or an industrial grade may also be used. Examples of the n-butene-containing gas that can be used include a residual component (raffinate 1) obtained by removing butadiene from a $C_4$ fraction produced as a byproduct in the thermal cracking of naphtha, a residual component (raffinate 2) obtained by removing isobutylene from raffinate 1, a butene fraction obtained by catalytic cracking of a heavy oil fraction, a butene fraction obtained by a dehydrogenation reaction or an oxidative dehydrogenation reaction of n-butane, a butene fraction obtained by dimerization of ethylene, and a $C_4$ fraction produced as a byproduct by a catalytic conversion reaction of ethylene obtained by the thermal cracking of ethane or a dehydrogenation reaction of biomass ethanol. Biomass ethanol is ethanol obtained from plant resources. Specific examples includes ethanol obtained by the fermentation of sugar cane, corn and the like, and ethanol obtained from ligneous resources, such as scrap wood, thinnings, rice straw, and crops.

From the viewpoint of butadiene productivity, it is preferred that the n-butene concentration in a raw material mixed gas is 2% by volume or more based on 100% by volume of the raw material mixed gas, and from the viewpoint of suppressing the burden on the catalyst, preferably 30% by volume or less. More preferably, the n-butene concentration in the raw material mixed gas is 3 to 25% by volume, and even more preferably 4 to 20% by volume.

The above-described raw material mixed gas is a gas that is fed to the reactor. The raw material mixed gas may be a mixture of a gas that includes n-butene and a gas that includes oxygen. In addition to n-butene and oxygen, the raw material mixed gas may also include, for example, unsaturated organic compounds, saturated organic compounds, aromatic organic compounds, water, steam, hydrogen, nitrogen, carbon dioxide, and carbon monoxide. Examples of unsaturated organic compounds include olefins, such as ethylene, propylene, isobutylene, pentene, hexene, heptene, and octene, as well as dienes. The above-described raffinate 2 may include a small amount (% by volume) of isobutylene. Examples of dienes include 1,3-butadiene and 1,2-butadiene. Examples of saturated organic compounds include saturated hydrocarbons, such as methane, ethane, propane, butane, pentane, hexane, heptane, octane, and nonane. Examples of aromatic organic compounds include aromatic hydrocarbons, such as benzene, toluene, and xylene. Further, after separating butadiene, which is the target product, from the reaction product, at least a portion of the unreacted n-butene may be recycled back to the fluidized bed reactor. Along with this recycling, a portion of the reaction byproducts may also be recycled back to the fluidized bed reactor. Examples of such reaction byproducts include aldehydes, carboxylic acids, ketones, and other oxygen-containing compounds. Specific examples include acetaldehyde, acrolein, methacrolein, benzaldehyde, acetic acid, acrylic acid, methacrylic acid, benzoic acid, maleic anhydride, phthalic acid, acetone, methyl vinyl ketone, and furan. Further, the raw material mixed gas may also include an acetylene, such as vinyl acetylene.

From an industrial viewpoint, it is in particular preferred to use air as the oxygen supply source. The oxygen concentration in the above-described raw material mixed gas can be represented in terms of air (with the concentration of oxygen in air considered to be 21% by volume) by "air/n-butene" as the volume ratio of air to n-butene. From the viewpoint of suppressing an excessive oxidation reaction, air/n-butene is preferably 1 to 25, more preferably 2 to 12, and even more preferably 3 to 10.

(2) Fluidized bed reaction (vapor-phase catalytic oxidation reaction)

First, the first step will be described. In the process for producing butadiene according to the present embodiment, the reaction apparatus includes at least a fluidized bed reactor (hereinafter, sometimes simply referred to as a "reactor"). The reactor, which includes in its interior as its main constituent parts a gas disperser, the internal contents, and a cyclone, has a configuration in which the raw material mixed gas is brought into contact with the catalyst while the catalyst is fluidized. As such a fluidized bed reactor, a fluidized bed reactor described in publicly-known document, for example, the Fluidized Bed Handbook (published by Baifukan Co., Ltd., 1999), may be used. It is preferred that the fluidized bed reactor is a bubbling fluidized bed reactor.

The fluidized bed reactor has nozzles that feed the n-butene-containing gas and nozzles that feed the oxygen-containing gas in its interior. The nozzles that feed the n-butene-containing gas are usually arranged higher than the nozzles that feed the oxygen-containing gas. The nozzles may be arranged so that the feed port of each gas is pointing either in an upward or a downward vertical direction.

The number of nozzles is, from the viewpoint of uniformly dispersing the bubbles of the raw material mixed gas over the catalyst layer, based on the area of the cross-section of the interior of the reactor orthogonal to the vertical direction, preferably 5 to 100 nozzles/m², more preferably 7 to 60 nozzles/m², and even more preferably 10 to 40 nozzles/m². It is preferred that the nozzles that feed the n-butene-containing gas and the nozzles that feed the oxygen-containing gas face each other in a 1:1 manner. However, as long as the number of nozzles is within the above range, the number of nozzles feeding the n-butene-containing gas may be greater than or less than the number of nozzles feeding the oxygen-containing gas. Further, the nozzles do not have to face each other. From the viewpoint of dispersing the n-butene-containing gas as uniformly as possible, it is preferred that the number of nozzles feeding the n-butene-containing gas is greater than the number of nozzles feeding the oxygen-containing gas.

The heat of reaction produced can be removed using a cooling pipe inserted into the reactor. Further, the product gas of this reaction can be temporarily exhausted from the reactor and fed to an absorption apparatus provided in the reaction apparatus or separately provided external to the reaction apparatus, components such as butadiene absorbed therein, and a portion of the remaining off-gases fed to the reactor.

It is preferred that the reactor in the present embodiment is connected by pipes to a below-described regenerator. These pipes are for removing the catalyst from the reactor to the regenerator, and returning the catalyst from the regenerator to the reactor in the below-described second step. It is preferred that the pipe for removing the catalyst (hereinafter, sometimes simply referred to as "removal pipe") has a connection part between the reactor and the removal pipe at a portion where the catalyst concentration in the reactor is high in order to efficiently remove the catalyst in the reactor. Further, it is preferred that a connection part between the reactor and a pipe for returning the catalyst (hereinafter, sometimes simply referred to as "return pipe") is provided at a portion where the catalyst concentration in the reactor is low in order to efficiently return the regenerated catalyst from the regenerator to the reactor.

The reaction temperature in the reactor is preferably in the range of 300 to 450°C, more preferably 320 to 420°C, and even more preferably 340 to 400°C. If the reaction temperature is 300°C or more, the conversion rate of the n-butene can be increased, and if the temperature is 450°C or less, the combustion decomposition rate of the produced butadiene can be suppressed more. Since the synthesis reaction of butadiene is an exothermic reaction, heat may be appropriately removed to obtain a preferred reaction temperature. In this case, the heat of reaction may be removed by a cooling pipe provided in the reactor. Further, in addition to this heat removal, the reaction temperature can also be regulated in the above range by supplying heat via a heating device.

From the viewpoint of further increasing the conversion rate of n-butene and the yield of butadiene, the reaction pressure in the reactor is preferably in the range of a slightly reduced pressure (e.g., -0.1 MPa/G) to 0.8 MPa/G, more preferably a normal pressure (0.0 MPa/G) to 0.4 MPa/G, and even more preferably 0.02 to 0.2 MPa/G. In the present specification, "MPa/G" means MPa in terms of gauge pressure.

Although the contact time between the raw material mixed gas and the catalyst cannot be definitively determined, in terms of the amount (g) of the catalyst in the reactor based on the flow rate of the raw material mixed gas (cc/sec) fed to the reactor, the contact time is preferably 0.1 to 20 (sec·g/cc), more preferably 1 to 10 (sec·g/cc), and in particular preferably 1 to 5 (sec·g/cc). If the contact time is in this range, butadiene, which is the target product, can be produced at a high yield. Further, the flow rate (cc/sec) of the raw material mixed gas is calculated in consideration of the temperature and pressure of the reaction conditions.

The gas including the produced butadiene (hereinafter, sometimes referred to as "butadiene-containing gas") is discharged from a reactor outlet. The oxygen concentration in the reactor outlet gas is preferably 0 to 3.0% by volume, more preferably 0.1 to 2.5% by volume, and even more preferably 0.2 to 2.0% by volume. The oxygen concentration in the reactor outlet gas can be adjusted by changing the amount of oxygen-containing gas fed to the reactor (e.g., the amount of air fed to the reactor), the reaction temperature, the pressure in the reactor, the amount of catalyst and the total amount of gasses fed to the reactor. By controlling the oxygen concentration in the reactor outlet gas to 3.0% by volume or less, combustion decomposition of the produced butadiene can be suppressed more, and the butadiene yield can be maintained at an even higher level.

(3) Purification

The reaction product obtained by a fluidized bed reaction can be purified by a publicly-known technique, for example, a method described in Japanese Patent Publication No. 45-17407, Japanese Patent Application Laid-Open No. 60-126235, and Japanese Patent Publication No. 3-48891, as well as a method described in Petrotech, Vol. 2. No. 4 (pages 59 to 65, 1978). If the butadiene concentration in the purified reaction product is 99% by volume or more, since the purified gas contains butadiene at a high purity, this gas can be preferably used as a raw material for synthetic rubber and the like.

(4) Catalyst regeneration

Next, the second step including the catalyst regeneration will be described. The process for producing butadiene according to the present embodiment includes a second step in which a part of the catalyst is removed from the fluidized bed reactor while the vapor-phase catalytic oxidation reaction is proceeding, the catalyst is regenerated in the regenerator under the predetermined conditions, and the regenerated catalyst is fed to the fluidized bed reactor while the reaction is proceeding. Here, the expression "while the reaction is proceeding" means a state in which the monoolefin having 4 carbon atoms is being fed to the fluidized bed reactor.

In addition, "stop the reaction" means stopping the feed of the monoolefin having 4 carbon atoms to the reactor.

In the present embodiment, the ratio of the amount (kg/hr) of the regenerated catalyst fed to the fluidized bed reactor to the amount (kg/hr) of the monoolefin having 4 carbon atoms fed to the fluidized bed reactor (hereinafter, sometimes referred to as "regeneration ratio") needs to be 0.3% or more. The regeneration ratio is preferably 0.7% or more, more preferably 1.5% or more, even more preferably 3.0% or more, and in particular preferably 4.0% or more. If the regeneration ratio is 0.3% or more, continuous operation over an extended period is possible. The higher the regeneration ratio, the greater the decrease in the amount of organic matter adhered to the catalyst in the reactor. Since the amount of organic matter adhered to the catalyst is inversely proportional to the butadiene yield, a higher regeneration ratio also enables the butadiene yield to be increased.

By removing the catalyst from the reactor in such a ratio, regenerating the catalyst, and feeding the regenerated catalyst to the reactor, the amount of organic matter adhered to the catalyst in the reactor can be kept roughly constant. Consequently, since there is no longer a need to stop the vapor-phase catalytic oxidation reaction used to produce butadiene during catalyst regeneration, butadiene can be stably produced at a high yield continuously over a long period. It is not necessary to adjust so that the amount of the catalyst removed from the reactor and the amount of the regenerated catalyst fed to the reactor are always equal, the ratio of these amounts may be freely changed. However, from the viewpoint of producing butadiene more stably, it is preferred to maintain the amount of the catalyst present in the fluidized bed reactor as constant as possible. From such a viewpoint, it is preferred to adjust so that the difference between the amount of the catalyst removed from the reactor and the amount of the regenerated catalyst fed to the reactor is small.

The greater the amount of the catalyst removed from the reactor and regenerated is, the lower the amount of organic matter adhered to the catalyst in the reactor tends to be. However, since there is a greater amount of the catalyst to be regenerated, more catalyst has to be regenerated within a predetermined time in the regenerator, so that the thermal load on the catalyst is increased as a result of increasing the temperature for regenerating the catalyst (hereinafter, sometimes simply referred to as "regeneration temperature") and the like. Consequently, degradation of the catalyst, such as sintering of the catalyst (decrease in the specific surface area) and changes in the crystal structure of the metal oxide, tends to be accelerated. Further, if the amount of the regenerated catalyst fed to the reactor is large, the normal state in the reactor tends to be impaired. Accordingly, from the viewpoint of preventing catalyst degradation, and from the viewpoint of maintaining the normal state in the reactor, it is preferred that the regeneration ratio is 100% or less, more preferably 60% or less, even more preferably 40% or less, and in particular preferably 20% or less.

Examples of the method for regenerating the catalyst removed from the fluidized bed reactor may include, but are not in particular limited to, heating the removed catalyst while flowing a gas that includes oxygen (hereinafter, sometimes simply referred to as "regeneration gas") through the regenerator.

It is preferred that the regenerator is, similar to the reactor, a fluidized type, namely, a fluidized bed regenerator. In the regenerator, it is preferred to regenerate the catalyst by feeding the regeneration gas from the bottom of the regenerator to burn off organic matter on the catalyst while making the catalyst on which organic matter is adhered flow, so that localized heat generation resulting from the combustion of the organic matter is suppressed and an excessive thermal load is not placed on the catalyst. Further, regeneration may also be carried out using a rotary furnace (rotary kiln) that is used to calcine the catalyst during the below-described production of the catalyst.

From the viewpoints of avoiding sudden combustion while efficiently burning off the organic matter adhered to the catalyst, and reducing the thermal load on the catalyst as much as possible, the regeneration temperature of the catalyst in the regenerator is preferably 250 to 550°C, more preferably 300 to 500°C, and even more preferably 350 to 450°C. Further, it is also preferred to gradually increase the temperature after feeding the catalyst to the regenerator.

The pressure in the regenerator, namely, the regeneration pressure, can be appropriately selected in consideration of the relationship with the pressure in the reactor, namely, the reaction pressure. Usually, it is preferred that the regeneration pressure is a slightly reduced pressure (e.g., -0.1 MPa/G) to 0.3 MPa/G, and more preferably 0.05 to 0.2 MPa/G.

From an industrial viewpoint, it is preferred that the supply source of the oxygen in the regeneration gas is air. From the viewpoint of avoiding sudden combustion of the organic matter adhered to the catalyst, it is preferred that the oxygen concentration in the regeneration gas is 0.5 to 21% by volume, more preferably 1 to 10% by volume, and even more preferably 1 to 5% by volume. Examples of the gas forming the portion other than oxygen in the regeneration gas include nitrogen ($N_2$), helium (He), water vapor ($H_2O$), and carbon dioxide ($CO_2$). Usually, this gas is $N_2$.

Although the contact time between the above-described regeneration gas and the catalyst during fluidized bed regeneration cannot be definitively determined, from the viewpoint of efficiently burning off the organic matter adhered to the catalyst, based on the ratio of the amount (g) of the catalyst in the regenerator to the flow rate of the regeneration gas (cc/sec) fed to the regenerator, the contact time is preferably 0.1 to 20 (sec·g/cc), and more preferably 1 to 10 (sec·g/cc). Further, the flow rate (cc/sec) of the regeneration gas is calculated in consideration of the temperature and pressure of the regeneration conditions.

The important points in this step of regenerating the catalyst are to reduce excessive thermal load on the catalyst by avoiding localized heat generation from the combustion of the organic matter, and not exposing the catalyst to a reducing atmosphere by ensuring that the oxygen concentration in the gas exhausted from the regenerator (hereinafter, referred to as "regenerator outlet gas") is 0.1% by volume or more. To satisfy this condition, in the regenerator it is preferred to appropriately combine the various conditions described above. For example, it is preferred to perform regeneration by feeding a regeneration gas having an oxygen concentration of 1 to 5% by volume so that the contact time with the catalyst is 1 to 5 (sec·g/cc), maintaining the regeneration pressure at 0.05 to 0.2 MPa/G, burning off the organic matter on the catalyst while gradually increasing the regeneration temperature to the range of 250 to 450°C, and maintaining the oxygen concentration in the regeneration outlet gas at 0.5% by volume or more.

Further, it is not necessary to completely remove the organic matter adhered to the catalyst. It is acceptable if a small amount remains.

The frequency of feeding the regenerated catalyst to the reactor is not in particular limited. The regenerated catalyst may be continuously fed or intermittently fed to the reactor, or fed based on a combination thereof. If the catalyst is continuously removed from the reactor, it is preferred to also continuously feed the regenerated catalyst to the reactor. On the other hand, if the catalyst is intermittently removed from the reactor, it is preferred to also intermittently feed the regenerated catalyst to the reactor. Further, in the case of intermittently feeding the regenerated catalyst, it is preferred to feed the regenerated catalyst to the reactor at least once every 15 days.

The amount of organic matter adhered to the catalyst in the reactor that is achieved by such regeneration is, based on the total amount of the catalyst and the adhered organic matter, preferably 10% by mass or less, more preferably 5% by mass or less, even more preferably 3% by mass or less, and in particular preferably 2% by mass or less. There is a correlation between the amount of organic matter adhered to the catalyst in the reactor and the butadiene yield, in which the lower the amount of organic matter, the higher the butadiene yield. Further, since the rate of adherence of organic matter to the catalyst decreases as the amount of organic matter adhered becomes smaller, from that viewpoint, it is preferred that the amount of organic matter adhered to the catalyst in the reactor is 3% by mass or less.

[2] Catalyst

(1) Structure

Although the catalyst is not in particular limited as long as it can be used in the process for producing butadiene, it is preferred that the catalyst includes a metal oxide containing molybdenum (Mo), bismuth (Bi), and iron (Fe). More preferably, the metal oxide is supported on a support. The composition ratio among the Mo, Bi, and Fe is adjusted so that a metal oxide that matches the intended purpose is formed in the catalyst. It is thought that an oxidative dehydrogenation reaction from n-butene to butadiene is carried out by using lattice oxygen in the metal oxide. Generally, if lattice oxygen in the catalyst is consumed by an oxidative dehydrogenation reaction, oxygen vacancies are produced in the metal oxide. These also cause reduction of the metal oxide as the reaction proceeds, so that catalytic activity is gradually decreased. Therefore, to maintain catalytic activity at a high level, it is necessary to quickly reoxidize the reduced metal oxide. A metal oxide that contains Mo, Bi, and Fe is not only active in an oxidative dehydrogenation reaction from n-butene to butadiene, it can also be considered that such a metal oxide will have excellent reoxidation characteristics for recovering consumed lattice oxygen by dissociatively adsorbing and incorporating molecular oxygen in the gas phase into the metal oxide. Therefore, from this viewpoint, the above catalyst is preferred.

If the catalyst including the metal oxide containing Mo, Bi, and Fe and the support supporting that metal oxide is used for producing butadiene by a fluidized bed process, there is an even greater effect in suppressing the secondary decomposition of butadiene, which is the product, and thus butadiene can be obtained at an even higher yield. Although the details are unclear, the reasons for this are thought to be (1) the fact that since the acidity of the catalyst is suitable, a secondary decomposition activity of butadiene on the catalyst is low, and (2) since the ability of the reaction sites of the catalyst to adsorb the produced butadiene is small, after the butadiene is produced, the butadiene quickly desorbs before undergoing decomposition at the reaction sites.

From the viewpoint of making it even easier to form the metal oxide that matches the intended purpose, it is preferred that the composition ratio among Mo, Bi, and Fe in the catalyst is, as an atom ratio p of the Bi and an atom ratio q of the Fe based on an Mo atom ratio of 12, $0.1 \leq p \leq 5$ and $0.5 \leq q \leq 8$.

In the catalyst, if the metal oxide contains metal elements other than Mo, Bi, and Fe, it is preferred that the metal oxide is represented by the following empirical formula (I).

$$Mo_{12}Bi_pFe_qA_aG_bJ_cL_dE_eO_x \qquad (I)$$

In this formula, A represents one or more elements selected from the group consisting of nickel and cobalt, G represents one or more elements selected from the group consisting of alkali metal elements, J represents one or more elements selected from the group consisting of magnesium, calcium, strontium, barium, zinc, and manganese, L represents one or more elements selected from the group consisting of rare earth elements, E represents one or more elements selected from the group consisting of chromium, indium, and gallium, O represents an oxygen atom, p, q, a, b, c, d, e, and x denote the atom ratio of bismuth, iron, A, G, J, L, E, and oxygen, respectively, based on 12 atoms of molybdenum, in which $0.1 \leq p \leq 5$, $0.5 \leq q \leq 8$, $0 \leq a \leq 10$, $0.02 \leq b \leq 2$, $0 \leq c \leq 5$, $0 \leq d \leq 5$, $0 \leq e \leq 5$, and x denotes the number of atoms of oxygen required to satisfy the valence requirement of the other elements that are present in the metal oxide.

In the present specification, the "empirical formula" represents a composition formed from an atom ratio of the metals included in this formula, and the required oxygen atoms based on that atom ratio and the sum of the metal oxidation numbers. In an oxide that includes metals capable of having several oxidation numbers, it is essentially impossible to specify the number of atoms of oxygen. Therefore, the oxygen number is formally denoted as "x". For example, for a metal oxide obtained by preparing a slurry that includes an Mo compound, a Bi compound, and a Fe compound, and drying and/or calcining this slurry, the atom ratio of the metals included in the slurry and the atom ratio of the metals in the obtained metal oxide can be considered to be essentially the same. Therefore, a formula that has $O_x$ added to the prepared composition formula of the slurry is the empirical formula of the obtained metal oxide. Further, in the present specification, like the prepared composition of the above-described slurry, since a formula representing the intentionally-controlled components and their ratios is called a "composition formula," in a case like that described above, the formula obtained by removing the $O_x$ from empirical formula (I) is the "composition formula."

Although the role of the components represented by A, G, J, L, and E is not limited, in the field of metal oxide catalysts having Mo, Bi, and Fe as essential components, the role of each component is presumed to be as follows. Namely, A and E are thought to improve the activity of the catalyst, G and J to stabilize the structure of the metal oxide including Mo, Bi, and Fe that matches the intended purpose, and L to have the effect of reoxidizing the metal oxide. If p, q, a, b, c, d, and e are within the above-described ranges, these effects can be expected to be substantially higher.

In the above-described empirical formula (I), a more preferred composition is $0.2 \leq p \leq 3.0$, $0.7 \leq q \leq 5.0$, $1.0 \leq a \leq 9.0$, $0.05 \leq b \leq 1.0$, $1.0 \leq c \leq 4.0$, $0.1 \leq d \leq 3.0$, and $0 \leq e \leq 4.0$. In an even more preferred composition, G represents one or more elements selected from the group consisting of rubidium, potassium, and cesium, J represents magnesium, L represents one or more elements selected from the group consisting of cerium, lanthanum, praseodymium, neodymium and yttrium, in which $0.3 \leq p \leq 2.0$, $1.0 \leq q \leq 3.0$, $2.0 \leq a \leq 8.0$, $0.1 \leq b \leq 0.5$, $1.5 \leq c \leq 3.5$, $0.2 \leq d \leq 2.0$, and $0 \leq e \leq 3.0$.

From the viewpoint of using the support more effectively, the content ratio of the support in the catalyst is, based on the total mass of the support and the metal oxide, preferably 30 to 70% by mass, more preferably 40 to 60% by mass, even more preferably 45 to 60% by mass, and in particular preferably 50 to 55% by mass. This catalyst including a metal oxide that contains Mo, Bi, and Fe can be obtained by a publicly-known method that includes, for example, step A of preparing a raw material slurry, step B of spray drying the raw material slurry, and step C of calcining the dried product obtained from step B. The support is preferably one or more selected from the group consisting of silica, alumina, titania, and zirconia, and a more preferred support is silica. Silica is a support that is more inert to an oxidative dehydrogenation reaction of n-butene than other supports. Silica also has a good binding action with the catalyst for obtaining butadiene, which is the target product, without reducing the activity or selectivity of the catalyst. Further, by supporting the metal oxide on the support, the catalyst can be provided with preferred physical properties (physical characteristics) for a fluidized bed reaction, such as particle shape, size, distribution, and fluidity, as well as mechanical strength.

In particular like in the present embodiment, if the catalyst is cycled between a reactor and a regenerator, the catalyst greatly needs to have a preferred particle shape, fluidity, and mechanical strength. An important factor in the control of these physical characteristics is the selection of the raw material of the above-described support. It is preferred to use the corresponding sol for the raw material of such supports. Namely, if the support includes silica, it is preferred to use a silica sol as the raw material, while if the support includes alumina, it is preferred to use an alumina sol as the raw material. Among these sols, by mixing two or more sols that have different average particle sizes for the oxide particles included therein, it tends to be easier to obtain a catalyst whose above-described physical characteristics are better. For example, if the support includes silica, it is preferred to produce the support from two or more silica sols having a different silica average particle size from each other. For example, the support can be produced from a silica sol having a silica average particle size of 10 to 15 nm and a silica sol having a silica average particle size of 30 to 50 nm. Such a support can obtain a catalyst having better particle shape and fluidity as well as better mechanical strength than when silica sols having the same silica average particle size are used alone. In addition, if a thus-obtained catalyst is used, the butadiene yield in the reaction producing butadiene from n-butene tends to be much better, and there tends to be less organic matter adhered to the catalyst during the butadiene production reaction. This is an advantageous effect that not even a person skilled in the art would have expected.

The average particle size of the silica or the like included in the sol can be measured by a nanoparticle size and distribution measurement apparatus (SALD-7100, manufactured by Shimadzu Corporation).

When mixing two or more sols which differ from each other in an average particle size of the oxide particles included therein, from the viewpoint of making the above-described physical characteristics of the catalyst even better, it is preferred that the difference in average particle size of the oxide particles between these sols is 5 to 40 nm, and more preferably 10 to 30 nm. For example, the average particle size of the oxide particles of the sol that includes the small oxide particles is preferably 5 to 20 nm, and the average particle size of the oxide particles of the sol that includes the large oxide particles is preferably 30 to 80 nm.

Further, when mixing two or more sols which differ from each other in an average particle size of the oxide particles included therein, from the viewpoint of more effectively and reliably obtaining the effects gained by mixing, it is preferred that the mixing ratio, as a mass ratio of the oxide particles between the sol including the oxide particles with a smaller average particle size and the sol including the oxide particles with a larger average particle size, is 95:5 to 5:95, and more preferable is 80:20 to 20:80.

(2) Production process

Although the process for producing the catalyst according to the present embodiment is not in particular limited, it is preferred to include step A of preparing a raw material slurry, step B of spray drying the raw material slurry, and step C of calcining the dried product obtained from step B. A preferred mode of the process for producing the catalyst will now be described.

In step A, a raw material slurry is obtained from the raw materials of the catalyst. Examples of the respective elements forming the catalyst include, as the elemental source of molybdenum, bismuth, iron, nickel, cobalt, alkali metal elements, magnesium, calcium, strontium, barium, zinc, manganese, rare earth elements, chromium, indium, and gallium, an ammonium salt, a nitrate, a hydrochloride, a sulfate, and an organic acid salt thereof that can dissolve in water or nitric acid. In particular, as the molybdenum source, an ammonium salt is preferred, and as the elemental source of bismuth, iron, nickel, an alkali metal, magnesium, zinc, manganese, and a rare earth element, a nitrate thereof is preferred. As described above, the support of the catalyst is preferably one or more selected from the group consisting of silica, alumina, titania, and zirconia, and a more preferred support is silica. As the silica source, a silica sol is more preferred. It is even more preferred to use as the silica source a mixture of two or more silica sols having a different silica average particle size from each other. Regarding impurities in the silica sol, it is preferred to use a silica sol having an aluminum content of not greater than 0.04 atoms for 100 silicon atoms, and more preferably a silica sol having an aluminum content of not greater than 0.02 atoms for 100 silicon atoms.

The raw material slurry can be obtained by an ordinary method. For example, the raw material slurry can be prepared by adding an ammonium salt of molybdenum dissolved in water to a silica sol, and then adding a solution in which a respective nitrate of bismuth, a rare earth element, iron, nickel, magnesium, zinc, manganese, and an alkali metal has been dissolved in water or aqueous nitric acid. During this process, the above-described order of addition may be changed.

In step B, the raw material slurry obtained from the above-described step A is spray dried to obtain spherical particles. Atomization of the raw material slurry can be performed based on a method that is usually carried out industrially, such as a centrifugal method, a two-fluid nozzle method, and a high-pressure nozzle method. Among these methods, a centrifugal method is preferably employed. Next, the obtained particles are dried. As the drying heat source, it is preferred to use air heated by steam, an electric heater and the like. The temperature at the dryer inlet is 100 to 400°C, and preferably 150 to 300°C.

In step C, the target catalyst is obtained by calcining the dry particles obtained from step B. If necessary, it is preferred to pre-calcine the dry particles at, for example, 150 to 500°C, and then calcine the particles for 1 to 20 hours at a temperature in the range of 500 to 700°C. The calcining can be carried out using a calcining furnace, such as a rotary furnace (rotary kiln), a tunnel furnace, and a muffle furnace. Although the average particle size of the obtained catalyst is not in particular limited, from the viewpoint of fluidity of the catalyst, the average particle size is preferably in the range of 40 to 70 $\mu$m, and more preferably in the range of 50 to 60 $\mu$m. Further, the particle size distribution of the catalyst is preferably such that the particle size of 90% by mass or more of the catalyst particles is in the range of 10 to 150 $\mu$m. The average particle size and the particle size distribution of the catalyst may be measured using the apparatus described in the following Examples.

Further, from the viewpoint of the fluidity of the catalyst, the angle of repose of the catalyst is preferably 15 to 40°, and more preferably 20 to 30°. From the viewpoint of suppressing pulverization of the catalyst in the fluidized bed reactor, it is preferred that the average compressive strength of the catalyst is 10 to 100 MPa, and 20 to 60 MPa is more preferred. The angle of repose and the average compressive strength may be measured using the apparatus described in the following Examples.

According to the present embodiment, a sudden increase in the amount of organic matter adhered to the catalyst that occurs with an oxidative dehydrogenation reaction of a monoolefin having 4 carbon atoms, such as n-butene, can be suppressed. Consequently, butadiene can be stably produced at a high yield continuously over a long period.

Examples

The present invention will now be described in more detail with reference to the following Examples. However, the present invention is not limited by the following Examples.

In the Examples and Comparative Examples, the n-butene conversion rate and the butadiene yield used to represent the reaction results are defined based on the following formulae.

$$\text{n-Butene conversion rate (\%)} = \text{(number of moles of reacted n-butene)}/\text{(number of moles of fed n-butene)} \times 100$$

$$\text{Butadiene yield (\%)} = \text{(number of moles of produced butadiene)}/\text{(number of moles of fed n-butene)} \times 100$$

A carbon steel reactor having an inner diameter of 0.6 m and a height of 17 m, and having inside the reactor a two-stage cyclone and a plurality of cooling pipes roughly evenly arranged in four stages in the height direction was used as a fluidized bed reactor. The feed of oxygen and an inert gas such as nitrogen (oxygen-containing gas) was carried out using seven nozzles (one in the center and six arranged at 60° intervals around the center at a distance of 200 mm) from a bottom portion of the reactor. The feed port of the n-butene-containing gas was arranged 300 mm above the feed port of the oxygen-containing gas, so that the n-butene-containing gas was fed so as to oppose the oxygen-containing gas. The reaction temperature was taken as an average value of measurement values obtained from four thermocouples arranged 0.25 m to 0.5 m from the bottom portion of the reactor. The gases produced from the reaction were analyzed using a gas chromatography apparatus (model number: GC2010 Plus, manufactured by Shimadzu Corporation).

[Measurement of the amount of organic matter adhered to the catalyst]

The catalyst was obtained from the reactor, and the amount of organic matter adhered to the catalyst was measured using a thermogravimetric analysis apparatus (model number: TG-8120, manufactured by Rigaku Corporation). The temperature was increased to 600°C while flowing an oxygen/helium mixed gas into the system, and the decrease in the mass of the catalyst to which organic matter had been adhered was measured. At 600°C, all of the organic matter had been burned off and removed. However, the decrease in mass up to 200°C was considered to be derived from adsorbed moisture, and was thus excluded.

(a) Catalyst preparation

[Catalyst A]

A catalyst in which a metal oxide with a composition represented by the empirical formula $Mo_{12}Bi_{0.60}Fe_{1.8}Ni_{5.0}K_{0.09}Rb_{0.05}Mg_{2.0}Ce_{0.75}O_x$ was supported on 50% by mass of silica based on the whole catalyst (total mass of the support and the metal oxide; hereinafter the same applies) was prepared as follows. A solution of 5.9 kg of bismuth nitrate [$Bi(NO_3)_3 \cdot 5H_2O$], 6.6 kg of cerium nitrate [$Ce(NO_3)_3 \cdot 6H_2O$], 14.7 kg of iron nitrate [$Fe(NO_3)_3 \cdot 9H_2O$], 29.3 kg of nickel nitrate [$Ni(NO_3)_2 \cdot 6H_2O$], 10.4 kg of magnesium nitrate [$Mg(NO_3)_2 \cdot 6H_2O$], 0.18 kg of potassium nitrate [$KNO_3$], and 0.15 kg of rubidium nitrate [$RbNO_3$] dissolved in 41.3 kg of 16.6% by mass of nitric acid was charged into 183.5 kg of a silica sol (silica average particle size of 12 nm, manufactured by Nalco Company) including 30% by mass of silica ($SiO_2$). The resulting mixture was further charged with a solution of 42.7 kg of para-ammonium molybdate [$(NH_4)_6Mo_7O_{24} \cdot 4H_2O$] in 86.1 kg of water to obtain a raw material preparation (step A). The obtained raw material preparation was fed to a co-current spray dryer, and dried at an inlet temperature of about 250°C and an outlet temperature of about 140°C. Atomization of the raw material preparation was carried out using an atomizer equipped with a dish-shaped rotor arranged in the center of an upper portion of the dryer (step B). The obtained powder was, in an electric rotary furnace, pre-calcined for 1 hour at about 350°C in an air atmosphere, and then calcined for 2 hours at 590°C in an air atmosphere (step C), whereby a catalyst was obtained. This catalyst preparation process was repeated to produce the necessary amount of catalyst A. The produced catalyst A had an average particle size of 50.5 $\mu$m (measured with an apparatus (Model name: MT 3000) manufactured by Microtrac; hereinafter the same applies), an angle of repose of 28° (measured with a cylinder rotary type angle of repose measurement device, manufactured by Tsutsui Scientific Instruments Co., Ltd.; hereinafter the same applies), and an average compressive strength of 24 MPa (measured with an apparatus (Model name: MCT) manufactured by Shimadzu Corporation; hereinafter the same applies).

[Catalyst B]

Catalyst B was prepared in the same manner as the preparation of catalyst A, except that a 70:30 (mass ratio) mixture of a sol having a silica average particle size of 12 nm and a sol having a silica average particle size of 40 nm was used as the silica sol. The obtained catalyst B had an average particle size of 50.2 $\mu$m, an angle of repose of 26°, and an average compressive strength of 35 MPa.

[Catalyst C]

Catalyst C was prepared in the same manner as the preparation of catalyst B, except that the catalyst included a metal oxide with a composition represented by the empirical formula $Mo_{12}Bi_{0.60}Fe_{1.2}Ni_{2.0}Co_{3.0}K_{0.09}Rb_{0.02}Mg_{2.0}Ce_{0.75}O_x$ supported on 50% by mass of silica based on the whole catalyst, in which a part of the raw material was changed to cobalt nitrate $[Co(NO_3)_2 \cdot 6H_2O]$, and the amount of each component was adjusted to match the above composition. The obtained catalyst C had an average particle size of 50.8 $\mu$m, an angle of repose of 26°, and an average compressive strength of 36 MPa.

[Catalyst D]

Catalyst D was prepared in the same manner as the preparation of catalyst B, except that the catalyst included a metal oxide with a composition represented by the empirical formula $Mo_{12}Bi_{1.2}Fe_{2.5}Co_{6.0}Mn_{2.2}La_{0.75}Cr_{0.02}O_x$ supported on 50% by mass of silica based on the whole catalyst, in which a part of the raw material was changed to cobalt nitrate $[Co(NO_3)_2 \cdot 6H_2O]$, manganese nitrate $[Mn(NO_3)_2 \cdot 6H_2O]$, lanthanum nitrate $[La(NO_3)_2 \cdot 6H_2O]$, and chromium nitrate $[Cr(NO_3)_2 \cdot 9H_2O]$, and the amount of each component was adjusted to match the above composition. The obtained catalyst D had an average particle size of 49.8 $\mu$m, an angle of repose of 26°, and an average compressive strength of 35 MPa.

[Catalyst E]

Catalyst E was prepared in the same manner as the preparation of catalyst A, except that a metal oxide with the same composition as catalyst A was supported on 50% by mass of alumina based on the whole catalyst. As the alumina source, an alumina sol manufactured by Nalco Company (trade name "Nalco 8676," alumina content: 10% by mass) was used. The obtained catalyst E had an average particle size of 51.0 $\mu$m, an angle of repose of 25°, and an average compressive strength of 55 MPa.

[Catalyst F]

Catalyst F was prepared in the same manner as the preparation of catalyst A, except that a metal oxide with the same composition as catalyst A was supported on a support formed from 43% by mass of silica and 7% by mass of titania based on the whole catalyst. As the silica-titania source, a silica-titania sol manufactured by Nalco Company (trade name "TX 11658," titania content: 4% by mass, silica content: 26% by mass) was used. The obtained catalyst F had an average particle size of 50.5 $\mu$m, an angle of repose of 26°, and an average compressive strength of 35 MPa.

[Catalyst G]

Catalyst G was prepared in the same manner as the preparation of catalyst A, except that a metal oxide with the same composition as catalyst A was supported on 50% by mass of zirconia based on the whole catalyst. As the zirconia source, a zirconia sol manufactured by Nalco Company (trade name "00SS008," zirconia content: 23% by mass) was used. The obtained catalyst G had an average particle size of 50.5 $\mu$m, an angle of repose of 27°, and an average compressive strength of 30 MPa.

(Example 1)

550kg of Catalyst A was charged into the above-described reactor, and a n-butene-containing gas (1-butene/2-butene/n-butene/other C4 hydrocarbons = 38/33/26/3 (volume ratio)) and an oxygen-containing gas (mixed gas of air and nitrogen) were oppositely fed to the reactor. The ratio of the respective gases included in the raw material gas was adjusted so that the n-butene concentration (total concentration of 1-butene and 2-butene) was 8% by volume, and air/n-butene was 4.7 (volume ratio). The amount of raw material mixed gas fed to the reactor was set at 7,093 NL/min. The contact time was adjusted to around 3.0 (g.sec/cc) so that the oxygen concentration at the reactor outlet was 1.0% by volume. Butadiene was produced by setting the reaction temperature to 360°C and the reaction pressure to 0.05 MPa/G (gauge pressure) (first step).

After carrying out the above reaction for 1.5 days, the catalyst in an amount of 25% by mass (137.5 kg) based on the total amount of the catalyst in the reactor was removed while performing the reaction to a regenerator via an removal pipe connecting the reactor and the regenerator. The removed catalyst was regenerated in the regenerator under the following conditions. All the catalyst that had finished regeneration was fed to the fluidized bed reactor while the reaction was proceeding via a return pipe connecting the regenerator and the reactor (until this point, second step). The reaction was continued even during this time. The amount of regenerated catalyst at this point was 3.82 kg/hr (i.e., 137.5 kg/36 hr = 3.82 kg/hr). Since the amount of the monoolefin having 4 carbon atoms (in this case, the amount of n-butene) in the raw material mixed gas fed to the reactor was 85.1 kg/hr (i.e., calculated from 7,093 NL/min $\times$ 0.08 (n-butene concentration) = 567.4 NL/min), the regeneration ratio was 4.5% (i.e., 3.82 (kg/hr)/85.1 (kg/hr) $\times$ 100 = 4.5%). In the following Examples 2 to 5 and 9 to 14, and Comparative Example 1, the regeneration ratio was calculated in the same manner.

In the regeneration of the catalyst, organic matter adhered to the catalyst was burned off by feeding an $O_2/N_2$ mixed gas ($O_2/N_2$ = 5/95% by volume) as the regeneration gas to the regenerator, while maintaining the regeneration pressure at 0.03 MPa/G (gauge pressure) and increasing the temperature from 250°C to 450°C over 5 hours. After that, the catalyst was held for further 7 hours at 450°C. The oxygen concentration at the regenerator outlet during this time was maintained at 1% by volume or more. It was confirmed that organic matter on the catalyst that had finished regeneration had been completely removed.

The operation as described above, in which regeneration was performed by intermittently removing the catalyst every 1.5 days while carrying out the reaction, and the regenerated catalyst was fed to the reactor, was continued for 50 days. During this period, it was possible to continue the operation in a stable manner. The reaction results and the amount of organic matter adhered to the catalyst in the reactor (amount of organic matter on the catalyst in the reactor) during this period are shown in Table 1.

(Example 2)

An operation in which the catalyst was intermittently removed every 1.5 days while carrying out the reaction, and regenerated, and the regenerated catalyst was fed to the reactor, was continued for 50 days in the same manner as in Example 1, except that catalyst B was used. During this period, it was possible to continue the operation in a stable manner. The reaction results during this period are shown in Table 1, and the trend in the amount of organic matter adhered to the catalyst in the reactor is illustrated in FIG. 1.

(Example 3)

An operation in which the catalyst was intermittently removed every 1.5 days while carrying out the reaction, and regenerated, and the regenerated catalyst was fed to the reactor, was continued for 50 days in the same manner as in Example 1, except that catalyst C was used. During this period, it was possible to continue the operation in a stable manner. The reaction results and the amount of organic matter adhered to the catalyst in the reactor during this period are shown in Table 1.

(Example 4)

the catalyst in an amount of 5% by mass (27.5 kg) based on the total amount of the catalyst in the reactor was intermittently removed every 4 days to the regenerator, and regenerated, and all of the regenerated catalyst was fed to the reactor. The amount of regenerated catalyst at this point was 0.286 kg/hr (i.e., 27.5 kg/96 hr = 0.286 kg/hr). Since the amount of the monoolefin having 4 carbon atoms (in this case, the amount of n-butene) in the raw material mixed gas fed to the reactor was 85.1 kg/hr, the regeneration ratio was 0.34% (i.e., 0.286 (kg/hr)/85.1 (kg/hr) $\times$ 100 = 0.34%). Apart from this, the operation was carried out in the same manner as in Example 2. During this period, it was possible to continue the operation in a stable manner. The reaction results and the amount of organic matter adhered to the catalyst in the reactor during this period are shown in Table 1, and the trend in the amount of organic matter adhered to the catalyst in the reactor is illustrated in FIG. 1.

(Example 5)

the catalyst in an amount of 25% by mass (137.5 kg) based on the total amount of the catalyst in the reactor was intermittently removed every 0.5 days to the regenerator, and regenerated, and the regenerated catalyst was fed to the reactor. The regeneration ratio at this point was 13.5%. Apart from this, the operation was carried out in the same manner as in Example 2. During this period, it was possible to continue the operation in a stable manner. The reaction results and the amount of organic matter adhered to the catalyst in the reactor during this period are shown in Table 1.

(Example 6)

Using catalyst B, the catalyst was continuously removed from the reactor to the regenerator, and regenerated. Specifically, from the start of reaction, the catalyst was continuously removed at 3.9 kg/hr from the reactor to the regenerator, and regenerated under the following conditions, and the regenerated catalyst was fed back into the reactor in the same ratio as the removed catalyst, so that the total amount of the catalyst in the reactor was constant. Apart from this, the reaction was carried out in the same manner as in Example 2. The regeneration ratio at this point was 4.6% (i.e., 3.9 (kg/hr)/85.1 (kg/hr) $\times$ 100 = 4.6%. In the following Examples 7, 8, 15, and 16, the regeneration ratio was calculated in the same manner).

In the regeneration of the catalyst, the above-described catalyst was placed in the regenerator maintained at 450°C. Regeneration was carried out by feeding an $O_2/N_2$ mixed gas ($O_2/N_2$ = 8/92 (% by volume)) as the regeneration gas to the regenerator, and maintaining the regeneration pressure at 0.05 MPa/G (gauge pressure). The oxygen concentration at the regenerator outlet was maintained at 1% by volume or more. The amount of organic matter adhered to the catalyst that had finished regeneration was 0.1% by mass.

This operation was carried out for 50 days, during which it was possible to continue the operation in a stable manner. The reaction results and the amount of organic matter adhered to the catalyst in the reactor during this period are shown in Table 1.

(Example 7)

From the start of reaction, the catalyst was continuously removed at 82.2 kg/hr from the reactor to the regenerator, and regenerated, and the regenerated catalyst was fed back into the reactor in the same ratio as the removed catalyst, so that the total amount of the catalyst in the reactor was constant. Apart from this, the operation was carried out in the same manner as in Example 6. The regeneration ratio at this point was 96.6%. This operation was carried out for 50 days, during which time it was possible to continue the operation in a stable manner. The reaction results and the amount of organic matter adhered to the catalyst in the reactor during this period are shown in Table 1, and the trend in the amount of organic matter adhered to the catalyst in the reactor is illustrated in FIG. 1.

(Example 8)

From the start of reaction, the catalyst was continuously removed at 0.60 kg/hr from the reactor to the regenerator, and regenerated, and the regenerated catalyst was fed back into the reactor in the same ratio as the removed catalyst, so that the total amount of the catalyst in the reactor was constant. Apart from this, the operation was carried out in the same manner as in Example 6. The regeneration ratio at this point was 0.71%. This operation was carried out for 50 days, during which time it was possible to continue the operation in a stable manner. The reaction results and the amount of organic matter adhered to the catalyst in the reactor during this period are shown in Table 1.

(Example 9)

An operation was carried out in the same manner as in Example 2, except that the n-butene-containing gas in Example 2 was changed to 1-butene, the 1-butene concentration in the raw material mixed gas was adjusted to 6% by volume, and the air/1-butene was adjusted to 4.9 (volume ratio). This operation was continued for 50 days, during which time it was possible to continue the operation in a stable manner. The reaction results and the amount of organic matter adhered to the catalyst in the reactor during this period are shown in Table 1.

(Example 10)

An operation in which the catalyst was intermittently removed every 1.5 days while carrying out the reaction, and regenerated, and the regenerated catalyst was fed to the reactor, was continued for 50 days in the same manner as in Example 1, except that catalyst D was used. During this period, it was possible to continue the operation in a stable manner. The reaction results during this period are shown in Table 1.

(Example 11)

An operation in which the catalyst was intermittently removed every 1.5 days while carrying out the reaction, and regenerated, and the regenerated catalyst was fed to the reactor, was continued for 50 days in the same manner as in Example 1, except that catalyst E was used. During this period, it was possible to continue the operation in a stable manner. The reaction results during this period are shown in Table 1.

(Example 12)

An operation in which the catalyst was intermittently removed every 1.5 days while carrying out the reaction, and regenerated, and the regenerated catalyst was fed to the reactor, was continued for 50 days in the same manner as in Example 1, except that catalyst F was used. During this period, it was possible to continue the operation in a stable manner. The reaction results during this period are shown in Table 1.

(Example 13)

An operation in which the catalyst was intermittently removed every 1.5 days while carrying out the reaction, and regenerated, and the regenerated catalyst was fed to the reactor, was continued for 50 days in the same manner as in Example 1, except that catalyst G was used. During this period, it was possible to continue the operation in a stable manner. The reaction results during this period are shown in Table 1.

(Example 14)

An operation was carried out in the same manner as in Example 2, except that the composition of the n-butene-containing gas was changed to 1-butene/2-butene/n-butane/iso-butene/1,3-butadiene/other C4 hydrocarbons = 47/32/13/3/1/4 (volume ratio). The amount of the regenerated catalyst at this point was 3.82 kg/hr (i.e., 137.5 kg/36 hr = 3.82 kg/hr). Since the amount of the monoolefin having 4 carbon atoms (in this case, the total amount of n-butene and iso-butene) in the raw material mixed gas fed to the reactor was 88.3 kg/hr (i.e., calculated from 7,093 NL/min $\times$ (0.08 (n-butene concentration) + 0.003 (iso-butene concentration) = 588.7 NL/min), the regeneration ratio was 4.3% (i.e., 3.82 (kg/hr)/88.3 (kg/hr) $\times$ 100 = 4.3%).
This operation was continued for 50 days, during which time it was possible to continue the operation in a stable manner. The reaction results and the amount of organic matter adhered to the catalyst in the reactor during this period are shown in Table 1.

(Example 15)

Using catalyst B, the catalyst was continuously removed from the reactor to the regenerator, and regenerated. Specifically, from the start of reaction, the catalyst was continuously removed at 103.8 kg/hr from the reactor to the regenerator, and regenerated under the following conditions, and the regenerated catalyst was fed back into the reactor in the same ratio as the removed catalyst, so that the total amount of the catalyst in the reactor was constant. Apart from this, the operation was carried out in the same manner as in Example 2. The regeneration ratio at this point was 122%.
In the regeneration of the catalyst, the above-described catalyst was placed in a regenerator maintained at 520°C. Regeneration was carried out by feeding an $O_2/N_2$ mixed gas ($O_2/N_2$ = 8/92 (% by volume)) as the regeneration gas to the regenerator, and maintaining the regeneration pressure at 0.05 MPa/G (gauge pressure). The oxygen concentration at the regenerator outlet was maintained at 1% by volume or more. Organic matter adhered to the catalyst that had finished regeneration had been completely removed.
This operation was carried out for 50 days. During this period, although the amount of organic matter adhered to the catalyst in the reactor was generally constant, an approximately 1% fluctuation in the n-butene conversion rate and the butadiene yield was observed. Although the exact reason for this phenomenon is unclear, it was thought to be due to the instability of the normal state in the reactor resulting from the large regeneration ratio and the frequent turnover of the catalyst in the reactor. The reaction results and the amount of organic matter adhered to the catalyst in the reactor during this period are shown in Table 1.

(Example 16)

Using catalyst B, the catalyst was continuously removed from the reactor to the regenerator, and regenerated. Specifically, from the start of reaction, the catalyst was continuously removed at 25.5 kg/hr from the reactor to the regenerator, and regenerated under the same conditions as in Example 15, and the regenerated catalyst was fed back into the reactor in the same ratio as the removed catalyst, so that the total amount of the catalyst in the reactor was constant. Apart from this, the reaction was carried out in the same manner as in Example 2. The regeneration ratio at this point was 30%. Organic matter on the catalyst that had finished regeneration had been completely removed. This operation was carried out for 50 days, during which time it was possible to continue the operation in a stable manner. The reaction results and the amount of organic matter adhered to the catalyst in the reactor during this period are shown in Table 1.

(Comparative Example 1)

The catalyst in an amount of 5% by mass (27.5 kg) based on the total amount of the catalyst in the reactor was intermittently removed every 7 days from the reactor, and regenerated, and the regenerated catalyst was fed to the reactor. The regeneration ratio at this point was 0.19%. Apart from this, the operation was carried out in the same manner as in Example 2. During this period, the amount of organic matter adhered to the catalyst in the reactor was not constant, and it gradually increased. Along with this, the butadiene yield also decreased, and the operation could not be stably continued. Consequently, the operation was stopped at day 42. The reaction results and the amount of organic matter adhered to the catalyst in the reactor during this period are shown in Table 1, and the trend in the amount of organic matter adhered to the catalyst in the reactor is illustrated in FIG. 1.

(Comparative Example 2)

The operation was carried out in the same manner as in Example 2, except that catalyst regeneration was not performed. The reaction was continued for 15 days, during which the amount of organic matter adhered to the catalyst in the reactor was not constant, and it gradually increased. Along with this, the butadiene yield also decreased, and the operation could not be stably continued. Consequently, the reaction was stopped at day 15. The reaction results and the amount of organic matter adhered to the catalyst in the reactor during this period are shown in Table 1, and the trend in the amount of organic matter adhered to the catalyst in the reactor is illustrated in FIG. 1. The amount of organic matter adhered to the catalyst tended to increase at an accelerated rate, whereby it was learned that adherence of new organic matter is promoted as the amount of organic matter adhered to the catalyst becomes greater.

As described above, based on a comparison between Examples 1 to 16 and Comparative Examples 1 and 2, it can be seen that the amount of organic matter that adheres to the catalyst can be generally suppressed at a roughly constant level if the ratio (regeneration ratio) of the amount (kg/hr) of the regenerated catalyst fed based on the amount (kg/hr) of the monoolefin having 4 carbon atoms fed is 0.3% or more. Consequently, it was found that butadiene could be stably produced at a high yield over a long period without having to stop the production process each time the catalyst is regenerated.

Further, from a comparison between Example 1 and Examples 2 and 3, it was found that when catalysts B and C (Examples 2 and 3), which were produced from silica sols having a different average particle size, were used as the support raw material, the amount of organic matter that adhered to the catalyst was less and the butadiene yield was higher than when using catalyst A (Example 1), which was produced from a silica sol having an identical average particle size. Further, it was also found that catalysts B and C had excellent properties as a fluidized bed catalyst, exhibiting a smaller angle of repose than catalyst A (the angle of repose is an index for the fluidity of the catalyst, in which a smaller angle of repose indicates better fluidity), and a higher strength.

[Table 1]

| | Catalyst | Regeneration ratio (%)* | 25th Day of Reaction | | |
|---|---|---|---|---|---|
| | | | Conversion Rate (%) | Yield (%) | Organic Matter (wt.%) |
| Example 1 | A | 4.5 | 90.2 | 72.2 | 2.5 |
| Example 2 | B | 4.5 | 90.5 | 79.5 | 1.4 |
| Example 3 | C | 4.5 | 90.2 | 78.9 | 1.5 |
| Example 4 | B | 0.34 | 91.0 | 77.0 | 3.0 |
| Example 5 | B | 13.5 | 90.0 | 79.8 | 0.6 |
| Example 6 | B | 4.6 | 90.3 | 79.5 | 1.4 |
| Example 7 | B | 96.6 | 90.5 | 80.2 | 0.2 |
| Example 8 | B | 0.71 | 91.0 | 77.0 | 2.4 |
| Example 9 | B | 6.0 | 97.4 | 81.6 | 0.8 |
| Example 10 | D | 4.5 | 90.3 | 75.5 | 2.4 |
| Example 11 | E | 4.5 | 90.0 | 69.5 | 3.5 |
| Example 12 | F | 4.5 | 90.2 | 70.2 | 3.0 |
| Example 13 | G | 4.5 | 90.3 | 70.0 | 3.2 |
| Example 14 | B | 4.3 | 90.1 | 77.0 | 1.4 |
| Example 15 | B | 122 | 89 (±1) | 78 (±1) | 0.1 |
| Example 16 | B | 30.0 | 90.5 | 80.0 | 0.4 |
| Comparative Example 1 | B | 0.19 | 88.6 | 74.5 | 6.5 |
| Comparative Example 2 | B | None | 88.0*** | 70.0*** | 12.0*** |

[Table 1]-continued

|  | 50th Day of Reaction | | |
|---|---|---|---|
|  | Conversion Rate (%) | Yield (%) | Organic Matter (wt.%) |
| Example 1 | 90.5 | 72.4 | 2.5 |
| Example 2 | 91.0 | 79.5 | 1.4 |
| Example 3 | 90.6 | 78.7 | 1.5 |
| Example 4 | 90.6 | 77.1 | 3.0 |
| Example 5 | 90.4 | 79.7 | 0.6 |
| Example 6 | 90.2 | 79.4 | 1.5 |
| Example 7 | 90.2 | 80.3 | 0.1 |
| Example 8 | 90.6 | 76.8 | 2.6 |
| Example 9 | 97.5 | 81.5 | 0.8 |
| Example 10 | 90.2 | 75.6 | 2.5 |
| Example 11 | 90.1 | 69.4 | 3.5 |
| Example 12 | 90.2 | 70.3 | 3.2 |
| Example 13 | 90.0 | 70.1 | 3.3 |
| Example 14 | 90.5 | 77.1 | 1.5 |
| Example 15 | 87 (±1) | 76 (±1) | 0.1 |
| Example 16 | 90.5 | 80.2 | 0.4 |
| Comparative Example 1 | 88.5** | 68.0** | 20.4** |
| Comparative Example 2 | - | - | - |

\* Regeneration ratio (%) = (amount [kg/hr] of regenerated catalyst fed to fluidized bed reactor)/(amount [kg/hr] of monoolefin having 4 carbon atoms in raw material mixed gas fed to fluidized bed reactor) × 100

\*\* Value at day 42 of reaction

\*\*\* Value at day 15 of reaction

Industrial Applicability

When producing butadiene by an oxidative dehydrogenation reaction by bringing a monoolefin having 4 carbon atoms into contact with a catalyst, butadiene can be stably produced at a high yield over a long period by performing based on a predetermined process an operation for regenerating the catalyst by burning off and removing organic matter adhered to the catalyst while performing the reaction. Therefore, the present invention has industrial applicability in the field of producing butadiene by an oxidative dehydrogenation reaction by bringing a monoolefin having 4 carbon atoms into contact with a catalyst.

**Claims**

1.    A process for producing butadiene, comprising:

a first step of producing butadiene by a vapor-phase catalytic oxidation reaction in the presence of a catalyst by feeding a monoolefin having 4 carbon atoms to a fluidized bed reactor; and

a second step of removing a part of the catalyst from the fluidized bed reactor while the vapor-phase catalytic oxidation reaction is proceeding, regenerating the catalyst removed from the fluidized bed reactor, and feeding the regenerated catalyst to the fluidized bed reactor while the vapor-phase catalytic oxidation reaction is proceeding,

wherein a ratio of an amount (kg/hr) of the regenerated catalyst fed to an amount (kg/hr) of the monoolefin having 4 carbon atoms fed is 0.3% or more.

2. The process for producing butadiene according to claim 1, wherein the ratio of the amount (kg/hr) of the regenerated catalyst fed to the amount (kg/hr) of the monoolefin having 4 carbon atoms fed is 0.3 to 100%.

3. The process for producing butadiene according to claim 1 or 2, wherein the catalyst comprises a metal oxide comprising at least Mo, Bi, and Fe.

4. The process for producing butadiene according to any one of claims 1 to 3, wherein the catalyst comprises a metal oxide represented by the following empirical formula (I),

$$Mo_{12}Bi_pFe_qA_aG_bJ_cL_dE_eO_x \qquad (I)$$

wherein A represents one or more elements selected from the group consisting of nickel and cobalt, G represents one or more elements selected from the group consisting of alkali metal elements, J represents one or more elements selected from the group consisting of magnesium, calcium, strontium, barium, zinc, and manganese, L represents one or more elements selected from the group consisting of rare earth elements, E represents one or more elements selected from the group consisting of chromium, indium, and gallium, $0.1 \leq p \leq 5$, $0.5 \leq q \leq 8$, $0 \leq a \leq 10$, $0.02 \leq b \leq 2$, $0 \leq c \leq 5$, $0 \leq d \leq 5$, $0 \leq e \leq 5$, and x denotes a number of atoms of oxygen required to satisfy a valence requirement of other elements that are present in the metal oxide.

5. The process for producing butadiene according to any one of claims 1 to 4, wherein the catalyst comprises a support, and the support comprises one or more selected from the group consisting of silica, alumina, titania, and zirconia.

6. The process for producing butadiene according to any one of claims 1 to 4, wherein the catalyst comprises a support, and the support is produced from two or more silica sols having a different silica average particle size from each other.

**Patentansprüche**

1. Verfahren zur Herstellung von Butadien, umfassend:

einen ersten Schritt der Herstellung von Butadien durch eine katalytische Dampfphasenoxidationsreaktion in Gegenwart eines Katalysators durch Zuführen eines Monoolefins, das 4 Kohlenstoffatome aufweist, in einen Wirbelschichtreaktor; und

einen zweiten Schritt des Entfernens eines Teils des Katalysators aus dem Wirbelschichtreaktor, während die katalytische Dampfphasenoxidationsreaktion voranschreitet, des Regenerierens des aus dem Wirbelschichtreaktor entfernten Katalysators und des Zuführens des regenerierten Katalysators zum Wirbelschichtreaktor, während die katalytische Dampfphasenoxidationsreaktion voranschreitet;

wobei das Verhältnis der Menge (kg/h) des zugeführten regenerierten Katalysators zur Menge (kg/h) des zugeführten Monoolefins, das 4 Kohlenstoffatome aufweist, 0,3% oder mehr beträgt.

2. Verfahren zur Herstellung von Butadien gemäß Anspruch 1, wobei das Verhältnis der Menge (kg/h) des zugeführten regenerierten Katalysators zur Menge (kg/h) des zugeführten Monoolefins, das 4 Kohlenstoffatome aufweist, 0,3% bis 100% beträgt.

3. Verfahren zur Herstellung von Butadien gemäß Anspruch 1 oder 2, wobei der Katalysator ein Metalloxid, das wenigstens Mo, Bi und Fe umfasst, umfasst.

4. Verfahren zur Herstellung von Butadien gemäß einem der Ansprüche 1 bis 3, wobei der Katalysator ein Metalloxid umfasst, das durch die folgende Summenformel (I) dargestellt wird:

$$Mo_{12}Bi_pFe_qA_aG_bJ_cL_dE_eO_x \qquad (I),$$

wobei A für ein oder mehrere Elemente steht, die aus der Gruppe ausgewählt sind, die aus Nickel und Kobalt besteht, G für ein oder mehrere Elemente steht, die aus der Gruppe ausgewählt sind, die aus Alkalimetallelementen besteht, J für ein oder mehrere Elemente steht, die aus der Gruppe ausgewählt sind, die aus Magnesium, Calcium, Strontium, Barium, Zink und Mangan besteht, L für ein oder mehrere Elemente steht, die aus der Gruppe ausgewählt sind, die aus Seltenerdelementen besteht, E für ein oder mehrere Elemente steht, die aus der Gruppe ausgewählt sind, die aus Chrom, Indium und Gallium besteht, $0,1 \leq p \leq 5$, $0,5 \leq q \leq 8$, $0 \leq a \leq 10$, $0,02 \leq b \leq 2$, $0 \leq c \leq 5$, $0 \leq d \leq 5$, $0 \leq e \leq 5$ und x eine Anzahl von Sauerstoffatomen bezeichnet, die erforderlich sind, um eine Valenzanforderung anderer Elemente, die in dem Metalloxid vorhanden sind, zu erfüllen.

5. Verfahren zur Herstellung von Butadien gemäß einem der Ansprüche 1 bis 4, wobei der Katalysator einen Träger umfasst und der Träger eines oder mehrere umfasst, die aus der Gruppe ausgewählt sind, die aus Siliciumoxid, Aluminiumoxid, Titanoxid und Zirconiumoxid besteht.

6. Verfahren zur Herstellung von Butadien gemäß einem der Ansprüche 1 bis 4, wobei der Katalysator einen Träger umfasst und der Träger aus zwei oder mehr Kieselsäuresolen hergestellt ist, die eine unterschiedliche mittlere Teilchengröße der Kieselsäure aufweisen.

## Revendications

1. Procédé de production de butadiène, comprenant :

   une première étape de production de butadiène par une réaction d'oxydation catalytique en phase vapeur en présence d'un catalyseur en introduisant une mono-oléfine contenant 4 atomes de carbone dans un réacteur à lit fluidisé ; et
   une seconde étape de retrait d'une partie du catalyseur à partir du réacteur à lit fluidisé tandis que la réaction d'oxydation catalytique en phase vapeur se poursuit, de régénération du catalyseur retiré à partir du réacteur à lit fluidisé, et d'introduction du catalyseur régénéré dans le réacteur à lit fluidisé tandis que la réaction d'oxydation catalytique en phase vapeur se poursuit,
   dans lequel un rapport entre une quantité (kg/heure) du catalyseur régénéré introduite et une quantité (kg/heure) de la mono-oléfine contenant 4 atomes de carbone introduite est de 0,3 % ou plus.

2. Procédé de production de butadiène selon la revendication 1, dans lequel le rapport entre la quantité (kg/heure) du catalyseur régénéré introduite et la quantité (kg/heure) de la mono-oléfine contenant 4 atomes de carbone introduite est de 0,3 % à 100 %.

3. Procédé de production de butadiène selon la revendication 1 ou 2, dans lequel le catalyseur comprend un oxyde métallique comprenant au moins Mo, Bi, et Fe.

4. Procédé de production de butadiène selon l'une quelconque des revendications 1 à 3, dans lequel le catalyseur comprend un oxyde métallique représenté par la formule empirique (I) suivante,

$$Mo_{12}Bi_pFe_qA_aG_bJ_cL_dE_eO_x \qquad (I)$$

dans laquelle A représente un ou plusieurs éléments sélectionnés dans le groupe constitué du nickel et du cobalt, G représente un ou plusieurs éléments sélectionnés dans le groupe constitué des éléments métalliques alcalins, J représente un ou plusieurs éléments sélectionnés dans le groupe constitué du magnésium, du calcium, du strontium, du baryum, du zinc, et du manganèse, L représente un ou plusieurs éléments sélectionnés dans le groupe constitué des éléments des terres rares, E représente un ou plusieurs éléments sélectionnés dans le groupe constitué du chrome, de l'indium, et du gallium, $0,1 \leq p \leq 5$, $0,5 \leq q \leq 8$, $0 \leq a \leq 10$, $0,02 \leq b \leq 2$, $0 \leq c \leq 5$, $0 \leq d \leq 5$, $0 \leq e \leq 5$, et x indique un nombre d'atomes d'oxygène nécessaire pour satisfaire une exigence de valence des autres éléments qui sont présents dans l'oxyde métallique.

5. Procédé de production de butadiène selon l'une quelconque des revendications 1 à 4, dans lequel le catalyseur comprend un support, et le support comprend un ou plusieurs sélectionnés dans le groupe constitué de la silice, de l'alumine, de l'oxyde de titane, et de la zircone.

6. Procédé de production de butadiène selon l'une quelconque des revendications 1 à 4, dans lequel le catalyseur comprend un support, et le support est produit à partir de deux ou plus de deux sols de silice présentant une taille moyenne de particule de silice différente les uns des autres.

**FIG. 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012046509 A
- JP 2012067047 A
- JP 201292092 B
- JP 4517407 B
- JP 60126235 A
- JP 3048891 A

**Non-patent literature cited in the description**

- Fluidized Bed Handbook. Baifukan Co., Ltd, 1999
- *Petrotech,* 1978, vol. 2 (4), 59-65